# EUROPEAN PATENT APPLICATION

(11) **EP 1 972 341 A1**
(43) Date of publication of application: **24.09.2008**
(21) Application number: 07104769.0
(22) Date of filing: 23.03.2007
(51) Int. Cl.: A61K 31/59, A61K 31/663, A61P 19/08, A61P 43/00

(54) **Pharmaceutical compositions comprising a bisphosphonate and vitamin D**

(71) Applicant: Novartis AG, 4056 Basel (CH)
(72) Inventor: The designation of the inventor has not yet been filed
(74) Representative: Leon, Susanna Iris

(57) **Abstract**

Parenteral pharmaceutical compositions comprising vitamin D, or a bisphosphonate and vitamin D for treatment or prevention of diseases of abnormally increased bone turnover and/or vitamin D deficiency.

## Description

The present invention relates to pharmaceutical compositions comprising a bisphosphonate and vitamin D and to their use in treating conditions of abnormally increased bone turnover.

Bisphosphonates are well known in the art as inhibitors of osteoclasts and for their ability to bind to hydroxyapatite crystals in bone. These compounds are therefore useful in the prevention and treatment of bone loss, for example in diseases such as Paget's disease, osteoporosis, osteopenia, metastatic bone disease, multiple myeloma, periodontal disease and tooth loss.

Bisphosphonates such as alendronate, pamidronate, risedronate, ibandronate and zoledronate have been shown to be effective bone resorption inhibitors and have been widely and successfully used in the treatment of a variety of bone-related diseases and disorders. However, bisphosphonate therapy has been associated with hypocalcemia. The serum calcium decrease can persist for some time following the treatment and can be prominent in vitamin D insufficient patients. As a result, adequate vitamin D and calcium intake is recommended for subjects on bisphosphonate therapy.

The primary role of vitamin D is to maintain calcium homoeostasis by increasing the efficiency of the intestine in absorbing dietary calcium and thereby helping ensure that the amount of calcium absorbed is adequate to maintain blood calcium in the normal range and to maintain skeletal mineralisation.

Furthermore, adequate serum levels of 25-hydroxyvitamin D are critical to maintaining the health and function of the immune, reproductive, muscular and skeletal systems in the human body. In most individuals, a large part of the circulating 25-hydroxyvitamin D originates from cholecalciferol or vitamin D3, which is synthesized in the skin upon exposure to UVB radiation, provided by sunlight: The vitamin D3 must undergo two separate hydroxylation steps to become activated in its primary biological role in calcium and phosphorus homeostasis. After its synthesis in the skin, it is transported to the liver where it is metabolized to 25-hydroxyvitamin D and may be stored or released into the circulation. This intermediary metabolite is the major circulating and storage form that is delivered to tissue for further activations and is the best measure of vitamin D sufficiency. When a physiological demand for calcium and phosphorus arises, circulating 25-hydroxyvitamin D is metabolised to its biologically active hormonal form, 1,25-dihydroxyvitamin D.

Currently there is no standard definition of optimal vitamin D status. However, growing scientific evidence links a deficiency in 25-hydroxyvitamin D to increased risk of osteoporosis, diabetes, cancer and autoimmune disorders. It has been recognized that even mild to moderate vitamin D deficiency may contribute to bone loss and muscle weakness, resulting in increased likelihood of falls and fractures. Clinical trials have shown that giving elderly patients a supplement of vitamin D and calcium resulted in a reduction in the hip fracture rate.

The availability of clinical assays of 25-hydroxyvitamin D has made possible the assessment of vitamin D needs in various patient groups., The prevalence of vitamin D insufficiency, also termed "hypovitaminosis D" or "sub-clinical vitamin D deficiency" is now believed to be much greater than previously estimated. Studies have indicated that there is a high global prevalence of vitamin D insufficiency. The amount of vitamin D produced by the skin upon exposure to sunlight is often inadequate, particularly during winter or due to extensive skin covering by clothing. Additional factors associated with inadequate vitamin D levels include lack of dietary vitamin D intake, nursing home environment and increasing age. Low serum vitamin D levels may lead to osteomalacia and myopathy, leading to falls and increased risk of fractures. 64% of postmenopausal women have low vitamin D levels, and vitamin D deficiency is even more prevalent in hip fracture patients. Achieving vitamin D repletion may reduce the risk of falls by 22%; falls constitute the largest single cause of injury mortality in elderly individuals.

Vitamin D supplementation is routinely given during clinical trials of bisphosphonates and is recommended on the respective product labels. The recommended daily allowance of vitamin D is somewhere between 400 and 800 IU. Typically, a patient on bisphosphonate therapy is given a daily oral supplement of 400-800 IU to be taken separately from the bisphosphonate. Nevertheless, vitamin D deficiency is widespread. In practice many patients fail to take the prescribed supplements; in one trial the compliance rate was only 61% after 6 months Also, physicians may overlook the need for the supplements. Furthermore, a daily oral vitamin D dose of 400 IU has been shown to be insufficient for fracture prevention. Even with a daily oral vitamin D supplement of at least 400 IU, 27% of North American women receiving osteoporosis therapy have vitamin D deficiency. Patients with depleted vitamin D stores need high dose supplementation of vitamin D for efficient repletion.

Surprisingly, despite the technical and clinical challenges, it has now been found that vitamin D may be administered parenterally in much higher doses than those stated by conventional guidelines, and that such administration achieves vitamin D repletion for a prolonged period of time without the need for frequent redosing. Yet more surprisingly, it has been found according to the invention that vitamin D, a fat-soluble compound, may be conveniently co-administered parenterally with a bisphosphonate. Bisphosphonates are hydrophilic and thus their co-administration with vitamin D in a single composition would not have been foreseen.

The invention in a first aspect provides a parenteral pharmaceutical composition comprising a bisphosphonate and vitamin D.

The invention in a second aspect provides a method of treating or preventing a condition of abnormally increased bone turnover in a mammal comprising simultaneous, separate or sequential parenteral administration of a bisphosphonate and vitamin D. Preferably the bisphosphonate and vitamin D are administered simultaneously, more preferably as a single composition comprising the two agents.

The invention in a third aspect provides the use of a bisphosphonate and vitamin D in the manufacture of a medicament for the treatment of a condition of abnormally increased bone turnover, the medicament being for parenteral administration.

The invention in a fourth aspect provides a kit comprising a pharmaceutical composition comprising a bisphosphonate and a pharmaceutical composition comprising vitamin D for simultaneous, separate or sequential parenteral administration.

The invention in a fifth aspect provides a kit comprising a pharmaceutical composition comprising a bisphosphonate and vitamin D and an information leaflet instructing that the composition be administered at a dosing interval of once per 6 months or less frequently.

The invention in a sixth aspect provides a parenteral pharmaceutical composition for administration by infusion at intervals of once per 6 months or less frequently, the composition comprising vitamin D in an amount of at least 100,000 IU.

The invention in a seventh aspect provides the use of vitamin D in the manufacture of a medicament for the treatment or prevention of vitamin D deficiency, wherein the medicament is a parenteral composition for infusion at intervals of once per 6 months or less frequently.

The invention in an eighth aspect provides a method of treating or preventing vitamin D deficiency comprising intermittently administering parenterally an effective amount of vitamin D, wherein the period between administrations is at least 6 months.

The bisphosphonates for use in the present invention are preferably N-bisphosphonates.

For the purposes of the present description an N-bisphosphonate is a compound which in addition to the characteristic geminal bisphosphate (P-C-P) moiety comprises a nitrogen containing side chain, e.g. a compound of formula I 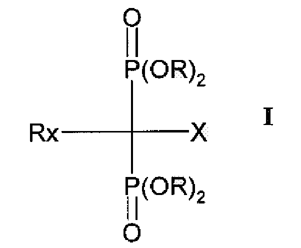 wherein
X is hydrogen, hydroxyl, amino, alkanoyl,or an amino group substituted by C₁-C₄ alkyl, or alkanoyl;
R is hydrogen or C₁-C₄ alkyl and
Rx is a side chain which contains an optionally substituted amino group, or a nitrogen containing heterocycle (including aromatic nitrogen-containing heterocycles),
and pharmaceutically acceptable salts thereof or any hydrate thereof.

Thus, for example, suitable N-bisphosphonates for use in the invention may include the following compounds or a pharmaceutically acceptable salt thereof, or any hydrate thereof: 3-amino-1-hydroxypropane-1,1-diphosphonic acid (pamidronic acid), e.g. pamidronate (APD); 3-(N,N-dimethylamino)-1-hydroxypropane-1,1-diphosphonic acid, e.g. dimethyl-APD; 4-amino-1-hydroxybutane-1,9-diphosphonic acid (alendronic acid), e.g. alendronate; 1-hydroxy-3-(methylpentylamino)-propylidene-bisphosphonic acid, ibandronic acid, e.g. ibandronate; 6-amino-1-hydroxyhexane-1,1-diphosphonic acid, e.g. amino-hexyl-BP; 3-(N-methyl-N-n-pentylamino)-1-hydroxypropane-1,1-diphosphonic acid; 1-hydroxy-2-{imidazol-1-yl}ethane-1,1-diphosphonic acid, e.g. zoledronic acid; 1-hydroxy-2-(3-pyridyl)ethane-1,1-diphosphonic acid (risedronic acid), e.g. risedronate, including N-methyl pyridinium salts thereof, for example N-methyl pyridinium iodides such as NE-10244 or NE-10446; 3-[N-(2-phenylthioethyl)-N-methylamino]-1 -hydroxypropane-1,1 1-diphosphonic acid; 1 -hydroxy-3-(pyrrolidin-1-yl)propane-1, 1-diphosphonic acid, e.g. EB 1053 (Leo); 1-(N-phenyl-aminothiocarbonyl)methane-1,1-diphosphonic acid, e.g. FR 78844 (Fujisawa); 5-benzoyl-3,4-dihydro-2H-pyrazole-3,3-diphasphonic acid tetraethyl ester, e.g. U-81 b81 (Upjohn); incadronate ((cycloheptylamino)methylene-bisphosphonate) and 1-hydroxy-2-(imidazo[1,2-a]pyridin-3-yl)ethane-1,1-diphosphanic acid, e.g. YM 529.

In one embodiment a particularly preferred N-bisphosphonate for use in the invention comprises a compound of Formula II 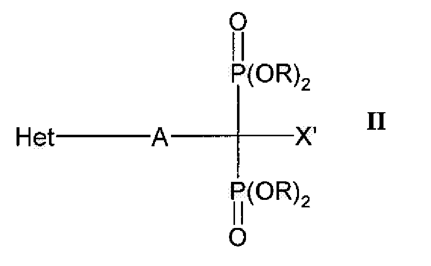 wherein
Het is an imidazole, oxazole, isoxazole, oxadiazole, thiazole, thiadiazole, pyridine, 1,2,3-triazole, 1,2,4'-triazole or benzimidazole radical, which is optionally substituted by alkyl, alkoxy, halogen, hydroxyl, carboxyl, an amino group optionally substituted by alkyl or alkanoyl radicals or a benzyl radical optionally substituted by alkyl, nitro, amino or aminoalkyl;
A is a straight-chained or branched, saturated or unsaturated hydrocarbon moiety containing from 1 to 8 carbon atoms;
X' is a hydrogen atom, optionally substituted by alkanoyl, or an amino group optionally substituted by alkyl or alkanoyl radicals, and
R is a hydrogen atom or an alkyl radical,
and the pharmacologically acceptable salts thereof.

In a further embodiment a particularly preferred bisphosphonate for use in the invention comprises a compound of Formula III 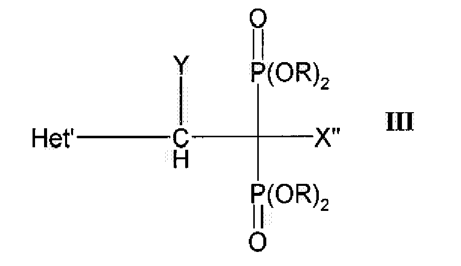 wherein
Het' is a substituted or unsubstituted heteroaromatic five-membered ring selected from the group consisting of imidazolyl, imidazolinyl, isoxazolyl, oxazolyl, oxazolinyl, thiazolyl, thiazolinyl, triazolyl, oxadiazolyl and thiadiazolyl wherein said ring can be partly hydrogenated and wherein said substituents are selected from at least one of the group consisting of C₁-C₄ alkyl, C₁₋C₄ alkoxy, phenyl, cyclohexyl, cyclohexylmethyl, halogen and amino and wherein two adjacent alkyl substituents of Het can together form a second ring;
Y is hydrogen or C₁-C₄ alkyl;
X" is hydrogen, hydroxyl, amino, or an amino group substituted by C₁-C₄ alkyl, and
R is hydrogen or C₁-C₄ alkyl;
as well as the pharmacologically acceptable salts and isomers thereof.

In a yet further embodiment a particularly preferred bisphosphonate for use in the invention comprises a compound of Formula IV 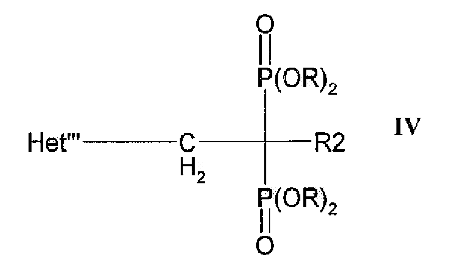 wherein
Het"' is an imidazolyl, 2H-1,2,3-, 1H-1,2,4- or 4H-1,2,4-triazolyl, tetrazolyl, oxazolyl, isoxazolyl, oxadiazolyl, thiazolyl or thiadiazolyl radical which is unsubstituted or C-mono-or di-substituted by lower alkyl, by lower alkoxy, bx phenyl which may in turn be mnon- or disubstituted by lower alkyl, lower alkoxy and/or halogen, by hydroxy, by di-lower alkylamino, by lower alkylthio and/or by halogen and is N-substituted at a substitutable N-atom by lower alkyl or by phenyl-lower alkyl which may in turn be mono- or di-substituted in the phenyl moiety by lower alkyl, lower alkoxy and/or halogen, and
R2 is hydrogen, hydroxy, amino, lower alkylthio or halogen,
lower radicals having up to and including 7 C-atoms,
or a pharmacologically acceptable salt thereof.

Examples of particularly preferred N-bisphosphonates for use in the invention are:
2-(1-Methylimidazol-2-yl)-1-hydroxyethane-1,1-diphosphonic acid;
2-(1-Benzylimidazol-2-yl)-1-hydroxyethane-1,1-diphosphonic acid;
2-(1-Methylimidazol-4-yl)-1-hydroxyethane-1,1-diphosphonic acid;
1-Amino-2-(1-methylimidazol-4-yl)ethane-1,1-diphosphonic acid;
1-Amino-2-(1-benzylimidazol-4-yl)ethane-1,1-diphosphonic acid;
2-(1-Methylimidazol-2-yl)ethane-1,1-diphosphonic acid;
2-(1-Benzylimidazol-2-yl)ethane-1,1-diphosphonic acid;
2-(Imidazol-1-yl)-1-hydroxyethane-1,1-diphosphonic acid;
2-(Imidazol-1-yl)ethane-1,1-diphosphonic acid;
2-(4H-1,2,4-triazol-4-yl)-1-hydroxyethane-1,1-diphosphonic acid;
2-(Thiazol-2-yl)ethane-1,1-diphosphonic acid;
2-(Imidazol-2-yl)ethane-1,1-diphosphonic acid;
2-(2-Methylimidazol-4(5)-yl)ethane-1,1-diphosphonic acid;
2-(2-Phenylimidazol-4(5)-yl)ethane-1,1-diphosphonic acid;
2-(4,5-Dimethylimidazol-1-yl)-1-hydroxyethane-1,1-diphosphonic acid, and
2-(2-Methylimidazol-4(5)-yl)-1-hydroxyethane-1,11-diphosphonic acid,
and pharmacologically acceptable salts thereof.

For example, the bisphosphonate may be alendronate, risedronate, ibandronate, minodronate, clodronate, etidronate, neridronate, olpadronate, tiludronate, pamidronate or zoledronate. The bisphosphonate may be provided as the free acid, e.g. alendronic acid, zoledronic acid, or as a salt or a hydrate, for example as alendronate monosodium trihydrate or zoledronic acid monohydrate or equilibrium mixtures of such salt, acid or hydrate.

The most preferred N-bisphosphonate for use in the invention is 2-(imidazol-1yl)-1-hydroxyethane-1,1-diphosphonic acid (zoledronic acid) or a pharmacologically acceptable salt thereof.

All the N-bisphosphonic acid derivatives mentioned above are well known from the literature. This includes their manufacture (see e.g. EP-A-513 760, pp. 13-48). For example, 3-amino-1-hydroxypropane-1,1-diphosphonic acid is prepared as described e.g. in US patent 3,962,432 as well as the disodium salt as in US patents 4,639,338 and 4,711,880, and 1-hydroxy-2-(imidazol-1-yl)ethane-1,1-diphosphonic acid is prepared as described e.g. in US patent 4,939,130. See also US patents 4,777,163 and 4,687,767.

The N-bisphosphonates may be used in the form of an isomer or of a mixture of isomers where appropriate, typically as optical isomers such as enantiomers or diastereoisomers or geometric isomers, typically cis-trans isomers. The optical isomers are obtained in the form of the pure antipodes and/or as racemates.

The N-bisphosphonates can also be used in the form of their hydrates or include other solvents used for their crystallisation.

The bisphosphonate in the composition is provided in an appropriate dose, for example, a composition of the invention may comprise a dose of 3-6 mg zoledronic acid, more preferably 4 mg or 5 mg.

The term "vitamin D" as used herein defines any active form of vitamin D, such as 25-dihydroxyvitamin D, or a metabolite thereof, or a non-active precursor thereof, for example 25-hydroxyvitamin D3 (25-hydroxycholecalciferol, calcifediol), vitamin D3 (cholecalciferol), vitamin D2 (ergocalciferol), 1αHydroxycholecalciferol (alfacalcidol), 1,25-dihydroxycholecalciferol (cacitriol), dihydroxytachysterol, doxercalciferol, provitamin D3 (7-dehydrocholesterol) and Vitamin D derivatives such as falecalcitriol, maxacalcitol and paricalcitol. In a preferred embodiment of the invention, the vitamin D is provided as a non-activated precursor to 25-hydroxyvitamin D, for example in the form of cholecalciferol or ergocalciferol. Yet more preferably, the vitamin D is provided as cholecalciferol.

In the present application 1 IU (international unit) of vitamin D3 is 0.025 micrograms of vitamin D3.

The vitamin D is present in an amount suitable to provide a subject with the daily need for vitamin D. The dosage provided will depend on the intended dosing interval of the composition. In preferred compositions for annual administration, the vitamin D is present in an amount of at least 10,000 IU, more preferably in an amount of at least 50,000, more preferably at least 100,000 IU. In more preferred compositions, vitamin D is provided in a dosage of at least 100,000 IU, more preferably at least 2DD,000 IU. For example, 300,000 or 600,000 IU of vitamin D is present in the composition. As a further example, a preferred composition of the invention may be a formulation comprising 300,000 IU of cholecalciferol. Such a formulation may be used for twice yearly or for annual administration or for even less frequent dosing, including a once only administration.

In a preferred embodiment, the invention comprises a parenteral' composition comprising zoledronic acid in a dose of 5mg and cholecalciferol in a dose of 200,000 - 600,000 IU.

The parenteral composition is preferably for administration by infusion.

The composition is advantageously provided as a formulation for infusion. This may be a pre-concentrate, to be diluted prior to administration, or a ready-to-use solution, fat emulsion or a dispersion. Alternatively, the composition may be provided as a lyophilisate, for example a lyophilized mixed micelle formulation. The formulation is preferably provided in a heat-sterilisable plastics container.

Alternatively, the composition may be in the form of a solid, for a example a lyophilisate, which can be used to prepare a formulation for infusion.

Alternatively, the composition may be a fat emulsion.

The parenteral composition may be administered at any suitable time interval, for example once daily, once weekly, once monthly, quarterly or at any time interval inbetween. Alternatively, the parenteral composition is administered at much greater time intervals of once per six months, once yearly, once every 18 months or at any time interval inbetween, or even less frequently, e.g. every 2-5 years, or even for once only dosing. Typically, the composition is for administration once every 3 months or less frequently. More preferably, it is for administration once per 6 months or less frequency. Yet more preferably the composition is for once yearly administration or less frequently. Alternatively, the composition is for once only dosing. In a particular preferred embodiment, the composition may be used for the treatment of Paget's disease, the composition being administered only once. The composition may on the other hand be readministered at a later time, in the event of a relapse as defined by symptoms and/or clinical assay.

Preferably the composition of the invention is administered by intravenous infusion over a period of at least 15 minutes.

Since bisphosphonates are hydrophilic whereas vitamin D is lipophilic, considerable difficulties arise in the preparation of a homogeneous, stable and pharmaceutically acceptable composition comprising the two agents. Certain excipients have been found to assist in the formulation of a stable and homogeneous formulation suitable for parenteral administration.

In a preferred embodiment, the parenteral composition of the invention is a formulation comprising ethoxylated esters of sorbitan with fatty acids (polysorbates)

In an alternative preferred embodiment, the parenteral composition of the invention is a mixed micelle formulation comprising phospholipid's and bile salts.

In an alternative preferred embodiment, the parenteral composition of the invention is a formulation comprising phospholipids as a solubiliser.

In another preferred embodiment, the parenteral composition is stabilized by the addition of antioxidants.

The compositions of the invention enable the convenient parenteral administration of vitamin D, and in certain embodiments the convenient parenteral co-administration of a bisphosphonate and vitamin D. The correct dose of each active is administered simultaneously and typically a further dose of either active is not needed for at least 3 months, typically at least 6 months or a year, or even longer. The single co-administration offers clear clinical benefits to the patient, who is replete in vitamin D for a prolonged, period of time without the need for frequent supplements which the patient might otherwise have difficulty in remembering to take, or be unwilling to do so. The invention therefore offers also the benefit of convenience and enhanced patient compliance.

As a result of vitamin D repletion, the patient benefits from increased muscle strength and consequentially a reduced incidence of falls.

Vitamin D is not normally administered in single doses as large 100,000 IU, nor as infrequently as in the present invention. Much lower doses on a more frequent basis, e.g. 400 IU once daily orally, are prescribed as a matter of course. The present invention is based on the unexpected finding that a large dose of vitamin D may be administered parenterally and that this has a prolonged duration of effect, with further doses not needed for long periods of time, for example 6 months or longer. Furthermore, a stable and pharmaceutically acceptable composition comprising a hydrophilic bisphosphonate and a lipophilic vitamin D would not have been envisaged as a feasible treatment option. The formulation of such a composition has presented considerable technical challenges.

Compositions of the invention may be used to treat or prevent conditions of abnormally increased bone turnover including: treatment of postmenopausal osteoporosis, e.g. to reduce the risk of osteoporotic fractures; prevention of postmenopausal osteoporosis, e.g. prevention of postmenopausal bone loss; treatment or prevention of male osteoporosis; treatment or prevention of corticosteroid-induced osteoporosis and other forms of bone loss secondary to or due to medication, e.g. diphenylhydantoin, thyroid hormone replacement therapy; treatment or prevention of bone loss associated with immobilisation and space flight; treatment or prevention of bone loss associated with rheumatoid arthritis, osteogenesis imperfecta, Paget's disease, hyperthyroidism, anorexia nervosa, organ transplantation, joint prosthesis loosening, and other medical conditions. For example, such other medical conditions may include treatment or prevention of periarticular bone erosions in rheumatoid arthritis; treatment of osteoarthritis, e.g. prevention/treatment of subchondral osteosclerosis, subchondral bone cysts, osteophyte formation, and of osteoarthritic pain, e.g. by reduction in intra-osseous pressure; treatment or prevention of hypercalcemia resulting from excessive bone resorption secondary to hyperparathyroidism, thyrotoxicosis, sarcoidosis, or hypervitaminosis D.

In the present description the terms "treatment" or "treat" refer to both prophylactic or preventative treatment as well as curative or disease modifying treatment, including treatment of patients at risk of contracting the disease or suspected to have contracted the disease as well as patients who are ill or have been diagnosed as suffering from a disease or medical condition. In particularly preferred embodiments the invention may be used for the prophylactic treatment of osteoporosis and similar diseases.

The compositions of the invention may also be used to treat conditions or procedures such as arthritic conditions, diseases which are associated with impairment of calcium metabolism, e.g. inflammatory conditions in joints, degenerative processes in articular cartilage, osteoporosis including post-menopausal osteoporosis, male osteoporosis and corticosteroid induced osteoporosis, osteopenia, periodontitis, hyperparathyroidism, calcium deposits in blood vessels or prosthetic implants; diseases in which an abnormal deposit of poorly soluble calcium salts is observed, as in arthritic disease, e.g. ankylosing spondylitis, neuritis, bursitis, periodontitis and tendonitis, fibrodysplasia, osteoarthrosis or arteriosclerosis; diseases in which an abnormal decomposition of hard body tissue is the principal symptom, e.g. hereditary hypophosphatasia, degenerative states of articular cartilage, osteoporosis of different provenance, Paget's disease, and osteodystrophia fibrosa, and also osteolytic conditions induced by tumours (i.e. bone metastases), multiple myeloma, tumor induced hypercalcemia, angiogenesis, cancer-treatment induced bone loss in patients who receive chemotherapy, hormone therapy or surgical castration, e.g. aromatase induced bone loss in breast cancer patients, or gonadotropin-releasing hormone agonists in prostate cancer,fracture healing , promotion of new bone at a fracture site, distraction osteogenesis, treatment and prevention of prosthesis loosening, osteonecrosis of the hip or knee, Perthes disease of the femoral head, pain, cancer-related pain, prostate cancer, treatment and prevention of recurrent fractures, atherosclerosis, stabilization of unstable plaques, cancer, myopathy, diseases of immune deficiency, gluten-sensitive enteropathy (non-tropical sprue), severe lactose intolerance, cirrhosis of any cause, severe right heart failure, short bowel syndrome and chronic giardiasis.

The invention is illustrated by way of the following non-limitative examples:

### Examples

### Example 1: Vitamin D / zoledronic acid formulation, 300'000 IU Vitamin D3

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.05 g |
| Cholecalciferol (Vitamin D3, cryst) = 300'000 IU | 0.0075 g |
| Water for Injections up to | 101.45 g = 100.0mL |

Vitamin D is dissolved in Ethanol, Polysorbate is added. Zoledronic acid is added to 80% of the calculated amount of water; sodium citrate and mannitol are added. Ethanolic solution of vitamin D is added under stirring to the aqueous phase. The solution is stirred until homogeneous. The solution is made up with water to the final weight.
The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.

### Stability:

| Test item | Start value | 3 months 5°C | 3months 25°C | 1 month 40°C/75% | 2 months 40°C/75% |
|---|---|---|---|---|---|
| Assay Vitamin D3, % of target | 100% | 99.3% | 100.4% | 98.4% | 97.6% |
| Particulate matter per 100mL >=10µm >=25µm | 240 7 | 147 0 | 460 53 | 227 0 | 620 7 |
| Sum of impurities corresponding to % of vitamin D3 | 2.11 | 1.52 | 2.25 | 2.02 | 2.75 |

### Example 2: Vitamin D / zoledronic acid formulation, 600'000 IU Vitamin D3

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.10g |
| Cholecalciferol (Vitamin D3, cryst) = 600'000 IU | 0.0150 g |
| Water for Injections up to | 101.45 g = 100.0mL |

Manufacture of the drug product is as described in Example 1.

**Example 3 and 4:** Vitamin D / zoledronic acid formulation, 300'000 and 600'000 IU Vitamin D3

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.05 g |
| Cholecalciferol (Vitamin D3, cryst) = 300'000 IU | 0.0075 g |
| α- Tocopherol | 0.050 g |
| Water for Injections up to | 101.45 g = 100.0mL |

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.10 g |
| Cholecalciferol (Vitamin D3, cryst) = 600'000 IU | 0.0150 g |
| α- Tocopherol | 0.050 g |
| Water for Injections up to | 101.45 g = 100.0mL |

Vitamin D and Tocopherol is dissolved in Ethanol, Polysorbate is added. Zoledronic acid is added to 80% of the calculated amount of water; sodium citrate and mannitol are added. Ethanolic solution of vitamin D is added under stirring to the aqueous phase. The solution is stirred until homogeneous. The solution is made up with water to the final weight.
The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.

### Example 5 and 6: Vitamin D / zoledronic acid formulation, 300'000 and 600'000 IU Vitamin D3

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.05 g |
| Cholecalciferol (Vitamin D3, cryst) = 300'00'0 IU | 0.0075 g |
| Butylated hydroxytoluene | 0.001 g |
| Water for Injections up to | 101.45 g = 100.0mL |

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.05 g |
| Cholecalciferol (Vitamin D3, cryst) = 600'000 IU | 0.0150 g |
| Butylated hydroxytoluene | 0.001 g |
| Water for Injections up to | 101.45 g = 100.0mL |

Vitamin D and Butylated hydroxytoluene is dissolved in Ethanol, Polysorbate is added. Zoledronic acid is added to 80% of the calculated amount of water; sodium citrate and mannitol are added. Ethanolic solution of vitamin D is added under stirring to the aqueous phase. The solution is stirred until homogeneous. The solution is made up with water to the final weight.

The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.

### Example 7: Vitamin D / zoledronic acid formulation, 300'000 IU Vitamin D3

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.20 g |
| Cholecalciferol (Vitamin D3, cryst) = 300'000 IU | 0.0075 g |
| Water for Injections up to | 101.45 g = 100.0mL |

Vitamin D is dissolved in Ethanol, Polysorbate is added. Zoledronic acid is added to 80% of the calculated amount of water; sodium citrate and mannitol are added. Ethanolic solution of vitamin D is added under stirring to the aqueous phase. The solution is stirred until homogeneous. The solution is made up with water to the final weight.
The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap. The final drug product is autoclaved for 15 minutes at >121°C

### Stability:

| Test item | Start value | 3 months 5°C | 3months 25°C | 1 month 40°C/75% | 2 months 40°C/75% |
|---|---|---|---|---|---|
| Assay Vitamin D3, % of target | 100% | 99.3% | 100.1% | 97.9% | 97.2% |
| Particulate matter per 100mL | | | | | |
| >=10µm | 1600 | 1160 | 2173 | 1027 | 1813 |
| >=25µm | 7 | 20 | 33 | 7 | 7 |
| Sum of impurities corresponding to % of vitamin D3 | 3.31 | 2.75 | 3.48 | 3.07 | 4.08 |

Analogous formulations containing Vitamin D2 instead of Vitamin D3 show less favorable stability.

### Example 8: Vitamin D / zoledronic acid formulation, 600'000 IU Vitamin D3

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.40 g |
| Cholecalciferol (Vitamin D3, cryst) = 600'000 IU | 0.0150 g |
| Water for Injections up to | 101.45 g = 100.0mL |

Manufacture of the drug product is as described in Example 7.

### Example 9 and 10: Vitamin D / zoledronic acid formulation, 300'000 and 600'000 IU Vitamin D3

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.20 g |
| Cholecalciferol (Vitamin D3, cryst) = 300'000 IU | 0.0075 g |
| α- Tocopherol | 0.050 g |
| Water for Injections up to | 101.45 g = 100.0mL |

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.40 g |
| Cholecalciferol (Vitamin D3, cryst) = 600'000 IU | 0.0150 g |
| α- Tocopherol | 0.050 g |
| Water for Injections up to | 101.45 g = 100.0mL |

Vitamin D and Tocopherol is dissolved in Ethanol, Polysorbate is added. Zoledronic acid is added to 80% of the calculated amount of water; sodium citrate and mannitol are added. Ethanolic solution of vitamin D is added under stirring to the aqueous phase. The solution is made up with water to the final weight.
The solution is filtered through a 0.2µm sterilizing filter and 100mL are filled into sterile 100mL cycloolefinic, polymer vials (Daikyo CZ). The vial is closed with fluroropolymer coated rubber stoppers (Helvoet FM2b9). The stopper is fixed with an aluminum crimp cap.
The final drug product is autoclaved for 15 minutes at >121°C

### Example 11 and 12: Vitamin D / zoledronic acid formulation, 300'000 and 600'000 IU Vitamin D3

| **Substance** | **Amount per vial** |
|---|---|
| Zoledronic acid H2O | D.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.20 g |
| Cholecalciferol (Vitamin D3, cryst) = 300'000 IU | 0.0075 g |
| Butylated hydroxytoluene | 0.001 g |
| Water for Injections up to | 101.45 g = 100.0mL |

| **Substance** | **Amount per vial** |
|---|---|
| Zoledronic acid H2O | 0.00533 g |
| equivalent to zoledronic acid anhydrous | 0.00500 g |
| Mannitol pyrogen free | 4.950 g |
| Sodium citrate | 0.030 g |
| Ethanol 94% | 0.50 g |
| Polysorbate 80 | 0.40 g |
| Cholecalciferol (Vitamin D3, cryst) = 600'000 IU | 0.0150 g |
| Butylated hydroxytoluene | 0.001 g |
| Water for Injections up to | 101.45 g = 100.0mL. |

Vitamin D and BHT is dissolved in Ethanol, Polysorbate is added. Zoledronic acid is added to 80% of the calculated amount of water; sodium citrate and mannitol are added. Ethanolic solution of vitamin D is added under stirring to the aqueous phase. The solution is made up with water to the final weight. The solution is stirred until homogeneous.

The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.
The final drug product is autoclaved for 15 minutes at >121°C

### Example 13: Vitamin D /zoledronic acid formulation with Lecithin as solubilizer, 300'000 IU Vitamin D3

| Substance | Amount/Vial |
|---|---|
| Zoledronic acid Monohydrate | 0.00533 g |
| Mannit pyrogenfree | 4.950 g |
| Sodium citrate | 0.030 g |
| Soy bean lecithin 70% PC | 0.10 g |
| LPOID S75 | |
| LIPOID GMBH | |
| Ethanol 94% m/m | 0.50 g |
| Cholecalciferol cryst. | 0.0075 g |
| Water for injections up to | 101.45 g = 100.0 mL |

Vitamin D and soy bean lecithin is dissolved in Ethanol. Zoledronic acid is added to 80% of the calculated amount of water, sodium citrate and mannitol are added. Ethanolic solution of vitamin D is added under stirring to the aqueous phase. The resulting turbid dispersion is stirred until homogeneous. The dispersion is made up with water to the final weight.

The dispersion is passed through a high pressure homogenizer (Avestin, EmulsiFlex-C5), (Vol. 100 ml in 5 /10 min) and a stable slightly yellow, slightly opalescent solution is formed. The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.

### Example 14: Vitamin D / zoledronic acid formulation with Lecithin as solubilizer, 300'000 and 600'000 IU Vitamin D3

| Substance | Amount/Vial (300'000 IU Vitamin D) | Amount/Vial (600'000 IU Vitamin D) |
|---|---|---|
| Zoledronic acid Monohydrate | 0.00533 g | 0.00533 g |
| Mannit pyrogenfree | 4.950 g | 4.950 g |
| Sodium citrate | 0.030 g | 0.030 g |
| Soy bean lecithin 70% PC | 0.10 g | 0.20 g |
| LPOID S75 | | |
| (LIPOID GMBH) | | |
| α- Tocopherol | 0.050 g | 0.050 g |
| Ethanol 94% m/m | 0.50 g | 0.50 g |
| Cholecalciferol cryst. | 0.0075 g | 0.0150 g |
| Water for injections up to | 101.45 g = 100.0 mL | 101.45 g = 100.0 mL |

Vitamin D, tocopherol and soy bean lecithin is dissolved in Ethanol. Zoledronic acid is added to 80% of the calculated amount of water, sodium citrate and mannitol are added. Ethanolic solution of vitamin D is added under stirring to the aqueous phase. The resulting turbid dispersion is stirred until homogeneous. The dispersion is made up with water to the final weight.

The dispersion is passed through a high pressure homogenizer (Avestin, EmulsiFlex-C5), (Vol. 100 ml in 5/10 min) and a stable slightly yellow, slightly opalescent solution is formed. The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.

### Example 15: Vitamin D / zoledronic acid formulation with Lecithin as solubilizer, 300'000 and 600'000 IU Vitamin D3

| Substance | Amount/Vial (300'000IU Vitamin D) | Amount/Vial (600'000 IU Vitamin D) |
|---|---|---|
| Zoledronic acid Monohydrate | 0.00533 g | 0.00533 g |
| Mannit pyrogenfree | 4.950 g | 4.950 g |
| Sodium citrate | 0.030 g | 0.030 g |
| Soy bean lecithin 70% PC | D.10 g | 0.20 g |
| LPOID S75 | | |
| (LIPOID GMBH) | | |
| Butylated hydroxytoluene | 0.001 g | 0.001 g |
| Ethanol 94% m/m | 0.50 g | 0.50 g |
| Cholecalciferol cryst. | 0.0075 g | 0.0150 g |
| Water for injections up to | 101.45 g = 100.0 mL | 101.45 g = 100.0 mL |

Vitamin D, BHT and soy bean lecithin is dissolved in Ethanol. Zoledronic acid is added to 80% of the calculated amount of water, sodium citrate and mannitol are added. Ethanolic solution of vitamin D is added under stirring to the aqueous phase. The resulting turbid dispersion is stirred until homogeneous. The dispersion is made up with water to the final weight.

The dispersion is passed through a high pressure homogenizer (Avestin, EmulsiFlex-C5), (Vol. 100 ml in 5/10 min) and a stable slightly yellow, slightly opalescent solution is formed. The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.

### Example 16: Vitamin D / zoledronic acid formulation with Lecithin as solubilizer, 300'000 and 600'000 IU Vitamin D3

| Substance | Amount/Vial (300'000 IU Vitamin D) | Amount/Vial (600'000 IU Vitamin D) |
|---|---|---|
| Zoledronic acid Monohydrate | 0.00533 g | 0.00533 g |
| Mannit pyrogenfree | 4.950 g | 4.950 g |
| Sodium citrate | 0.030 g | 0.030 g |
| Soy bean lecithin 70% PC | 0.10 g | 0.20 g |
| LPOID S75 | | |
| (LIPOID GMBH) | | |
| Ascorbyl palmitate | 0.01 g | 0.01 g |
| Ethanol 94% m/m | 0.50 g | 0.50 g |
| Cholecalciferol cryst. | 0.0075 g | 0.0150 g |
| Water for injections up to | 101.45 g = 100.0 mL | 101.45 g = 100.0 mL |

Vitamin D, ascorbylpalmitate and soy bean lecithin is dissolved in Ethanol. Zoledronic acid is added to 80% of the calculated amount of water, sodium citrate and mannitol are added Ethanolic solution of vitamin D is added under stirring to the aqueous phase. The resulting turbid dispersion is stirred until homogeneous. The dispersion is made up with water to the final weight.
The dispersion is passed through a high pressure homogenizer (Avestin, Emulsiflex-C5), (Vol. 100 ml in 5 /10 min) and a stable slightly yellow, slightly opalescent solution is formed. The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.

### Example 17: Vitamin D / zoledronic acid formulation with Lecithin as solubilizer, 300'000 and 600'000 IU Vitamin D3

| Substance | Amount/Vial (300'000 IU Vitamin D) | AmountNial (600'000 IU Vitamin D) |
|---|---|---|
| Zoledronic acid Monohydrate | 0.00533 g | 0.00533 g |
| Mannit pyrogenfree | 4.950 g | 4.950 g |
| Sodium citrate | 0.030 g | 0.030 g |
| Soy bean lecithin 70% PC | 0.10 g | 0.20 g |
| LPOID S75 | | |
| (LIPOID GMBH) | | |
| Butylated hydroxyanisol | 0.0003 g | 0.0003 g |
| Ethanol 94% m/m | 0.50 g | 0.50 g |
| Cholecalciferol cryst. | 0.0075 g | 0.0150 g |
| Water for injections up to | 101.45 g = 100.0 mL | 101.45 g = 100.0 mL |

Vitamin D, butylated hydrioxyanisol and soy bean lecithin is dissolved in Ethanol. Zoledronic acid is added to 80% of the calculated amount of water, sodium citrate and mannitol are added. Ethanolic solution of vitamin D is added under stirring to the aqueous phase. The resulting turbid dispersion is stirred until homogeneous. The dispersion is made up with water to the final weight.
The dispersion is passed through a high pressure homogenizer (Avestin, EmulsiFlex-C5), (Vol. 100 ml in 5 /10 min) and a stable slightly yellow, slightly opalescent solution is formed.
The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.

### Example 18: Vitamin D / zoledronic acid formulation in a mixed micelle formulation, 300'000 and 600'000 IU Vitamin D3

| Substance | Amount/Vial (300'000 IU Vitamin D3) | AmountNial (600'000 IU Vitamin D3) |
|---|---|---|
| Zoledronic acid Monohydrate | 0.00533 g | 0.00533 g |
| Mannit pyrogenfree | 4.950 g | 4.950 g |
| Sodium citrate | 0.030 g | 0.030 g |
| Sodium desoxycocholate | 0.20 g | 0.40 g |
| Soy bean lecithin 70% PC | 0.02 g | 0.04 g |
| LPOID S75 | | |
| (LIPOID GMBH) | | |
| Ethanol 94% m/m | 0.50 g | 0.50 g |
| Vitamin D3 99% | 0.0075 g | 0.0150 g |
| Water for injections up to | 101.45 g = 100.0 mL | 101.45 g = 100.0 mL |

Vitamin D and soy bean lecithin is dissolved in Etanol. Zoledronic acid is added to 80% of the calculated amount of water, sodium citrate, mannitol and sodium desoxycholic acid are added. Ethanolic solution of vitamin D is added under stirring to the aqueous phase. The solution is stirred until homogeneous. The dispersion is made up with water to the final weight.
The solution is filtered through a 0.2µm sterilizing filter and 100mL are filled into sterile 100mL cycloolefinic polymer vials (Daikyo CZ). The vial is closed with fluroropolymer coated rubber stoppers (Helvoet FM259). The stopper is fixed with an aluminum crimp cap.

### Example 19: Vitamin D / zoledronic acid formulation in a lyophilized mixed micelle formulation, 300'000 and 600'000 IU Vitamin D3

| Substance | Amount/Vial (300'000 IU Vitamin D3) | Amount/Vial (600'000 lU Vitamin D3) |
|---|---|---|
| Zoledronic acid Monohydrate | 0.00533 g | 0.00533 g |
| Mannit pyrogenfree | 0.50 g | 0.50 g |
| Sodium citrate | 0.030 g | 0.030 g |
| Sodium desoxycocholate | 0.20 g | 0.40 g |
| Soy bean lecithin 70% PC | 0.02 g | 0.04 g |
| LPOID S75 | | |
| (LIPOID GMBH) | | |
| Ethanol 94% m/m | 0.50 g | 0.50 g |
| Vitamin D3 99% | 0.0075 g | 0.0150 g |
| Water for injections up to | 5.0 mL | 5.0 mL |

Vitamin D and soy bean lecithin is dissolved in Ethanol. Zoledronic acid is added to 80% of the calculated amount of water, sodium citrate, mannitol and sodium desoxycholic acid are added. Ethanolic solution of vitamin D is added under stirring to the aqueous phase. The solution is stirred until homogeneous.
The solution is filtered through a 0.2µm sterilizing filter and 5mL of the solution are filled into a sterile and pyrogen free 100R tubing glass vial. A Daikyo D21-7S Fluorotec coated lyophilization rubber stopper is placed to the lyophilization position on the vial. The vials are lyophilized according to a preprogrammed cycle ensuring that the residual moisture is below 4%. The lyophilizer chamber is backflushed with pure Nitrogen up to slightly below atmospheric pressure (e.g. 900 mbar), and the stoppers are pressed into the vials to their final position within the lyophilizer. Outside the lyophilizer the stoppers are secured with an aluminum crimp cap.

The lypophilised composition may be prepared for administration by addition of 100 ml of a 4.5% mannitol solution or ca 0.8% NaCl.

### Example 20: Vitamin D / zoledronic acid formulation in a parenteral fat emulsion, 300'000 and 600'000 IU Vitamin D3

| Substance | Amount/Vial (300'000 IU Vitamin D3) | Amount/Vial (600'000 IU Vitamin D3) |
|---|---|---|
| Zoledronic acid Monohydrate | 0.00533 g | 0.00533 g |
| Glycerol | 2.50 g | 2.5 g |
| Sodium citrate | 0.030g | 0.030 g |
| Soy bean lecithin 70% PC | 0.4 g | 0.4 g |
| LPOID S75 | | |
| (LIPOID GMBH) | | |
| Purified soy bean oil | 3.00 g | 3.00 g |
| Vitamin D3 99% | 0.0075 g | 0.0150 g |
| αTocopherol acetate | 0.05 g | 0.05 g |
| Water for injections up to | 100.0 mL | 100.0 mL |

Vitamin D, soy bean lecithin and tocopherol acetate are dissolved in soy bean oil. Zoledronic acid is added to the calculated amount of water, sodium citrate and glycerol are added.The oily solution of vitamin D is added under stirring with a rotor stator stirrer (e.g. Ultra-Turrax) to the aqueous phase.
The dispersion is passed through a high pressure homogenizer (Avestin, EmulsiFlex-C5), (Vol. 100 ml in 10 -15 min) and a stable white emulsion is formed.
The emulsion is filtered through a 5µm filter and 100mL are filled into sterile 100mL cycloolefinic polymer vials (Daikyo CZ). The vial is closed with a D21-7S Fluorotec coated rubber stopper, and the stopper is secured with an aluminum crimp cap. The emulsion may be sterilized by autoclaving (121 °C/15 Min).

The formulations prepared according to each of the above examples may be stored in a heat-sterilisable plastics container, e.g. an infusion bag.

### Example 21: Use of composition for the treatment of osteoporosis

A formulation according to any one of Examples 1-20 is administered to a patient in need thereof by i.v. infusion over a period of 15 minutes. The infusion is repeated after a period of approximately one year.

### Example 22: Use of composition for the treatment of Paget's disease

A formulation according to any one of Examples 1-20 is administered to a patient in need thereof by i.v. infusion over a period of 15 minutes. The infusion is repeated if necessary, typically after a period of 2-5 years or longer, dependent on a clinical assessment of the condition of the patient, e.g. serum alkaline phosphatase levels measured in the patient and/or symptoms of the patient.

## Claims

1. A parenteral pharmaceutical composition comprising a bisphosphonate and vitamin D.

2. A composition according to claim 1 wherein the bisphosphonate is zoledronic acid.

3. A composition according to any one of the preceding claims wherein the vitamin D is cholecalciferol or ergocalciferol.

4. A composition according to any one of the preceding claims wherein the composition is a solution for infusion.

5. A composition according to any one of the preceding claims wherein the composition is for administration at dosing intervals of once per 6 months or less frequently.

6. A composition according to any one of the preceding claims wherein the composition is for administration at dosing intervals of once per year or less frequently.

7. A composition according to any one of the preceding claims wherein the composition is for administration at dosing intervals dependent on the results of an assay of the patient's blood.

8. A composition according to any one of the preceding claims wherein the vitamin D is cholecalciferol present in a dosage of at least 100,000 IU.

9. A composition according to any one of the preceding claims wherein the vitamin D is cholecalciferol present in a dosage of at least 300,000 IU.

10. A composition according to any one of the preceding claims wherein the bisphosphonate is zoledronic acid and is provided in a dosage of 3-6 mg.

11. A composition according to any one of the preceding claims wherein the composition comprises phospholipids.

12. A composition according to any one of the preceding claims wherein the composition comprises an antioxidant.

13. A composition according to claim 12 wherein the composition comprises tocopherol as antioxidant.

14. A composition according to claim 12 wherein the composition comprises butylated hydroxytoluene as antioxidant.

15. A composition according to claim 12 wherein the composition comprises butylated hydroxyanisol as antioxidant.

16. A composition according to claim 12 wherein the composition comprises ascorbyl palmitate as antioxidant.

17. A composition according to any one of the preceding claims wherein the composition comprises lecithin.

18. A composition according to claim 17 wherein the lecithin comprises less than 80% phosphatidyl choline.

19. A composition according to any one of the preceding claims wherein the composition is a mixed micelle formulation.

20. A composition according to claim 17 comprising desoxycholic acid as bile salt.

21. A composition according to claim 17 comprising glycocholic acid as bile salt.

22. A composition according to claim 17-21 wherein lecithin with a content of at least 80% is used.

23. A method of treating or preventing a condition of abnormally increased bone turnover in a mammal comprising simultaneous, separate or sequential parenteral administration of a bisphosphonate and vitamin D.

24. A method according to claim 23 comprising administration of a composition according to any one of claims 1-22.

25. Use of a bisphosphonate and vitamin D in the manufacture of a medicament for the treatment of a condition of abnormally increased bone turnover, the medicament being for parenteral administration.

26. A kit comprising a pharmaceutical composition comprising a bisphosphonate and vitamin D and an information leaflet instructing that the composition be administered at a dosing interval of once per 6 months or less frequently for the treatment of a condition of abnormally increased bone turnover.

27. A kit comprising a pharmaceutical composition comprising a bisphosphonate and a pharmaceutical composition comprising vitamin D for simultaneous, separate or sequential parenteral administration.

28. A method of treating or preventing vitamin D deficiency comprising intermittently administering parenterally an effective amount of vitamin D, wherein the period between administrations is at least 6 months.

29. A parenteral pharmaceutical composition for intermittent administration by infusion at intervals of once per 6 months or less frequently, the composition comprising vitamin D in an amount of at least 100,000 IU.

30. Use of vitamin D in the manufacture of a medicament for the treatment or prevention of vitamin D deficiency, wherein the medicament is a parenteral composition for infusion at intervals of once per 6 months or less frequently.
